# EUROPEAN PATENT APPLICATION

(11) **EP 0 673 658 A1**
(43) Date of publication of application: **27.09.1995**
(21) Application number: 95103615.1
(22) Date of filing: 02.04.1991
(51) Int. Cl.: A61M 1/14

(54) **System for controlling a medical treatment, for example dialysis**

(30) Priority: 25.05.1990 SE 9001890
(62) Divisional of application: 91105180.3
(71) Applicant: GAMBRO AB, S-220 10 Lund (SE)
(72) Inventor: Felding, Anders, S-216 18 Malmö (SE)
(74) Representative: Asketorp, Göran

(57) **Abstract**

Medical treatment apparatus, such as a dialysis monitor, comprising a vessel (1',1'',1''') containing a cleaning agent, such as citric acid, connected to a supply means (1a) for a solvent, for introducing the solvent into the vessel (1',1'',1''') and further to the medical treatment apparatus for disinfection and/or sterilization thereof. The medical treatment apparatus comprises a recirculation circuit including a heating device (2) for heating the cleaning fluid.

## Description

### AREA OF INVENTION

The present invention relates to a medical treatment apparatus, for example an apparatus for dialysis, using a preferably heated treatment fluid, comprising conditioning means for preparing and controlling the fluid and flowing it through a flow path and connection means for connecting the apparatus to a treatment device, for example a dialyzer. Cleaning of the apparatus is effected with a cleaning fluid which is made to flow along the same path as the treatment fluid, except for said treatment device.

Preferably, the apparatus according to the invention is intended for preparation of dialysis fluid in connection with haemodialysis. With minor modifications it can also, however, be used for preparation of replacement fluid in connection with haemofiltration or haemodiafiltration. It will be evident to the skilled man that the apparatus can also be used in connection with many other medical treatment methods where a treatment fluid is utilized or such a fluid is produced, for example wound rinsing fluid.

### PRIOR ART

A treatment fluid based on bicarbonate was originally used for dialysis. When the systems and monitors for this were later automated, difficulties arose, amongst them, precipitation. Other treatment fluids were therefore used instead, for example fluids based on acetate. Recently bicarbonate-based liquids have again found favour, at the same time the mentioned problems have in the main been overcome. However, certain problems still remain regarding precipitation. Thus, apparatuses in use must be cleaned at regular intervals. Hitherto this has been achieved by rinsing the systems with the aid of a cleaning liquid, for example citric acid.

US Patent No. 4 728 496 discloses an apparatus and a method for sterilization of, by way of example, a dialysis monitor. According to this system a fluid such as water is recirculated and heated within a part of the apparatus preceding the dialyzer. As is explained in the following, this apparatus can, after certain amendments, also be used for the application of the present invention.

US Patent No. 4 784 495 discloses a system for preparing a fluid intended for a medical procedure, for example dialysis, wherein the treatment fluid is prepared from a concentrate in powder form, for example sodium bicarbonate. Even in this system the present invention can be advantageously applied.

US Patent No. 4 789 467 discloses an automated disinfection system for a hemodialysis machine, having a container filled with a disinfection solution. A coupling tip is provided for connection to a check valve forming a part of hemodialysis machine. The liquid disinfection solution is introduced in the ordinary treatment path of the system.

### DISCLOSURE OF THE INVENTION

An object of the present invention is to provide a medical treatment apparatus in which cleaning by a cleaning agent, such as a chemical agent, can be performed in a simple manner.

Another object of the present invention is to obtain disinfection and/or sterilization of said medical treatment apparatus by combined heat disinfection and chemical disinfection.

A further object of the present invention is to obtain effective cleaning of a part of the medical treatment apparatus preceding the treatment device.

A still further object of the invention is to provide a medical treatment apparatus in which the cleaning agent is prvoided in a convenient way in a vessel or cartridge comprising the cleaning agent in a quantity sufficient for one cleaning operation.

Still further objects appear from the ensuing description.

Accordingly, there is provided a medical treatment apparatus intended for preparation and/or administration of a treatment fluid, for example an apparatus for dialysis. The apparatus comprises an inlet for introducing a fluid, preferably essentially water, into a conduit of the apparatus, conditioning means for preparing and/or controlling said fluid for forming said treatment fluid and/or for flowing said treatment fluid through a flow path of said apparatus; connection means for connecting the apparatus to a treatment device, for example a dialyzer, for supplying said treatment fluid to said treatment device; and introduction means for introducing a cleaning fluid into said apparatus and for flowing said cleaning fluid along essentially the same flow path as the treatment fluid, except for said treatment device, for cleaning the apparatus.

According to the invention the apparatus comprises a vessel containing a cleaning agent in concentrated liquid or powder form, which achieves or at least aids said cleaning, said vessel being connected to supply means for supplying a solvent to said vessel whereby said solvent enters the vessel for mixing with the cleaning agent for diluting and/or dissolution of said cleaning agent for forming said cleaning fluid and the vessel also being connected to the introduction means. The vessel comprises an inlet for introducing said solvent into said vessel and an outlet for connection to said introduction means. Preferably, the supply means for supplying a solvent is a means for supplying water.

In a preferred embodiment, the apparatus comprises recirculation means for recirculating at least a portion of the cleaning fluid through a recirculation path comprising a heating device for increasing and essentially maintaining the temperature of the cleaning fluid at a predetermined temperature, for example a little over 90°C, and flow means for flowing the cleaning fluid thorugh a non-recirculation path, wherein the temperature of the cleaning fluid is allowed to gradually decrease to a lower predetermined value, for example 80°. Preferably, the non-recirculation path is a portion of said flow path downstreams of said connection means.

In the preferred embodiment, the vessel comprises the cleaning agent in a quantity sufficient for one cleaning operation. The cleaning agent can be citric acid, peracetic acid, oxalic acid, sodium hydroxide, sodium hypochloride, sodium carbonate or suitable combinations thereof.

### BRIEF DESCRIPTION OF DRAWINGS

Further objects and advantages appear from the following description of a preferred embodiment of the invention with reference to the drawings.

Fig. 1 is a schematic view of an apparatus designed according to the invention.

Fig. 2 is a cross-sectional view showing means for connecting a concentrate cartridge or other vessel to the apparatus according to Fig. 1.

### BEST MODE FOR CARRYING OUT THE INVENTION

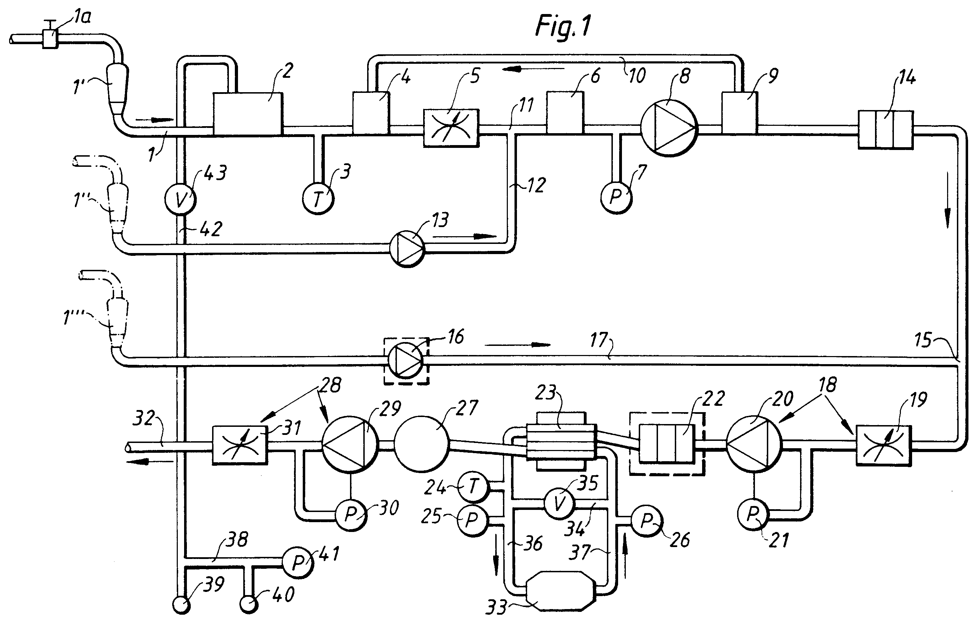

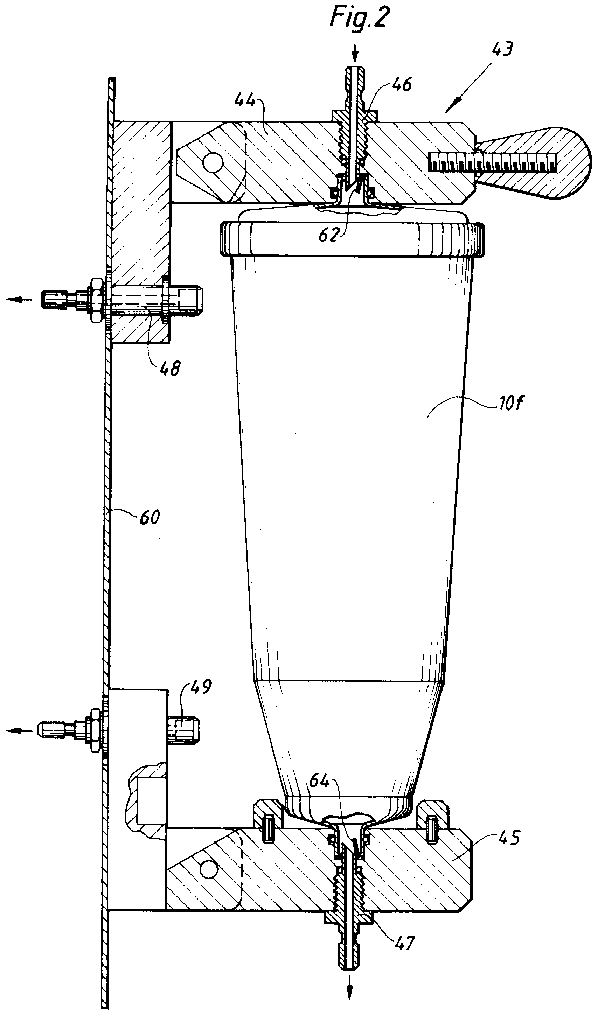

Fig. 1 shows a preferred embodiment of a system or apparatus according to the invention. In the system water is supplied via an inlet 1a through a water conduit 1 to a heating device 2 for heating. In Fig. 1 three various possible connection positions 1', 1'' respectively 1''' are shown for the above mentioned cartridge or other vessel containing a cleaning or cleansing concentrate. The same cartridge is shown in Fig. 2 with reference 10f together with means for its connection.

Water is taken from the heating device 2 with or without cleansing concentrate via a temperature measuring device 3, a return vessel 4, a throttle 5, a bubble-expansion chamber 6, a pressure measuring device 7 and a pump 8 to a ventilating chamber 9. A return conduit 10 leads from the ventilating chamber 9 back to the return vessel 4 for returning separated air or other gases together with a small quantity of fluid. A return line to the heating vessel 2 could instead be provided, but this would, however, require the use of chemically resistant material, since dialysis concentrate is normally supplied to the point 11 via conduit 12 with the help of the pump 13. This part of the system corresponds in the main with the system which is described by way of example in US Patents Nos. 4 158 034 and 4 293 409. The operation of the expansion chamber 6 is described in more detail in US Patent No. 4 536 201. Fluid is led from the ventilating chamber 9 via a conductivity measuring cell 14 to a further mixing point 15 where any additional concentrate is supplied with the aid of the pump 16 and a conduit 17. This is on the assumption that a so called two-component-based dialysis concentrate is to be used, for example of the type which is described in EP-B-O 022 922. Dialysis fluid is led from the mixing point 15 through a first constant flow device 18 comprising a throttle 19, a pump 20 and a pressure measuring device 21.

The pressure measured by the pressure measuring device 21 is used for controlling the pump 20 so that a desired constant flow is achieved. After the pump 20 the flow is led through a conductivity measurer 22 and ultra-filtration control 23 via a temperature measurer 24 and a pressure measurer 25 to a dialyzer 33. From there the flow is normally led via a pressure measurer 26, said ultra-filtration control 23 and a blood detector 27 to a further constant flow device 28 comprising a pump 29, a pressure measurer 30 and a throttle device 31. Finally, the dialysate is led to an outlet 32.

The construction and function of the ultra-filtration control is described in more detail in GB-B-2 003 274 and EP-B-0 106 940. Figure reference numeral 33 denotes the dialyzer connectable to the system according to the invention, to whose blood-side the patient is connected. This latter connection is, however, not shown in Figure 1.

The dialyzer 33 can be by-passed in two ways. This can occur either with the aid of by-pass conduit 34 with valve 35, which opens, for example if the temperature or the conductivity exceeds or falls below predetermined level. At the same time, the flow to the dialyzer is interrupted with the help of a not shown valve.

Alternatively, the by-pass can occur by means of the dialyzer connections 36 and 37 being connected to a by-pass conduit 38 via connections 39 and 40. This by-pass arrangement is principally designed according to US Patent No. 4 122 010 with a pressure monitoring device 41 which detects if a positive or negative pressure arises in conduit 38. If such is the case, and only then, sterilizing and/or cleansing can take place. Thus, treatment operation and cleansing operation can not be confused.

In the shown example a return conduit 42 with a valve 43 extends from the by-pass arrangement 38 back to the heating vessel 2. A quantity of the flow recirculates through this conduit 42 when the system is to be disinfected and/or sterilized and/or cleansed in any other way. The remainder of the flow is instead led from conduit 36 which is connected to connector 39 via conduit 38 and connector 40 to the conduit 37 and from there through the ultra-filtration control 23 and further to the outlet 32. A smaller quantity is, however, led directly from the conduit 36 via the conduit 34 with the valve 35 directly to the conduit 37 for cleansing of the by-pass connection.

According to the invention a cartridge or other vessel containing a cleansing concentrate is connected at 1', 1'' or 1'''. An example of such a connection is shown in Figure 2. The cartridge consists here of a closed vessel 10f which is provided at its ends with penetrable membranes 62 and 64. In this way, the cartridge is completely sealed so that the cleansing concentrate is not contaminated and can not escape the cartridge until the membranes are penetrated.

The cartridge 10f and means for its connection to the system according to the invention can be designed totally in accordance with the above mentioned US Patent No. 4 784 495. The differance is solely that the cartridge shall contain a cleansing concentrate instead of a treatment concentrate. The cartridge 10f is thus connected to the system with the aid of penetrating nippels 46 and 47 which are arranged on two lever arms 44 and 45. Fluid is supplied via nippel 46 and removed via nippel 47. The idea behind the lever arms 44 and 45 is that the system can also be used without a cartridge. The lever arms are swung to a position such that the nippels 46 and 47 are connected instead to a by-pass conduit connected to two nippels 48 and 49. These nippels, like the lever arms 44 and 45, are fixed to the wall 60 of a not shown control-monitor, for example such as used for controlling dialysis.

Examples of applicable cleansing agents can be: citric acid, acetic acid, peracetic acid, oxalic acid, sodium hydroxide, sodium hypochloride, sodium carbonates or suitable combinations thereof, though preferably citric acid. When citric acid is used, a suitable solution is obtained when 40 grams of citric acid is dissolved in two litres of water to a concentration of two per cent.

Alternatively, the citric acid can be "spiked" with oxalic acid. In this way any iron and copper precipitations are dissolved. By way of example a water solution can be used containing sixteen per cent citric acid and four per cent oxalic acid. This gives a very effective cleansing. A further alternative is the use of a water solution comprising 150 g/l sodium hypochloride, 4 g/l sodium hydroxide and 10-25 g/l of sodium carbonate which gives an effective cleansing after suitable dilution (approx. 20 times).

The above-mentioned solutions can be prepared in advance. Preferably, however, they are prepared directly in the system since the connected cartridge contains the concentrates in powder form.

Many advantages are obtained with the invention. In addition to the above-mentioned advantages, no mixing of the cleansing solution is needed in the clinic or pharmacy. Instead this can take place directly in the system. The possibility of using powder concentrate offers weight-saving advantages. However, not all concentrates need to be in powder form, since it is important that the invention can also be used with the aid of liquid-based concentrates.

The utilized concentrate cartridge can be connected at the beginning of the cleansing program and remain so during the whole of the rinsing program, which always follows the cleansing. This makes handling simpler. Cleansing agents which are harmless for the patient can be used. For example, citric acid is a basic food stuff and, as such, any small remnents are harmless for the patient. An important advantage is also the possibility to use various cleansing agents suited to respective treatment systems and conditions of use. The function of the system itself is also safer through effective cleansing. For dialysis, the necessary program change in existing programs is very slight.

Naturally, the invention is not restriced to the above described examples, but can be varied within the scope of the appended claims. For example, the shown details can be varied within wide limits of form and function.

## Claims

1. A method of cleaning a medical treatment apparatus intended for preparation and/or administration of a treatment fluid, for example an apparatus for dialysis, said apparatus comprising:
an inlet (1a) for introducing a fluid, preferably essentially water, into a conduit (1) of the apparatus,
conditioning means (2 - 22) for preparing and/or controlling said fluid for forming said treatment fluid and/or for flowing said treatment fluid through a flow path of said apparatus;
connection means (36,37) for connecting the apparatus to a treatment device (33), for example a dialyzer, for supplying said treatment fluid to said treatment device; and
introduction means (1,8;12,13;16,17) for introducing a cleaning fluid into said apparatus and for flowing said cleaning fluid along essentially the same flow path as the treatment fluid, except for said treatment device (33), for cleaning the apparatus;
**characterized** by
supplying a solvent to a vessel (1', 1'', 1''') containing a cleaning agent in concentrated liquid or powder form for mixing with said cleaning agent for diluting and/or dissolving said cleaning agent for forming said cleaning fluid;
introducing said cleaning fluid via said introduction means into said medical treatment apparatus for achieving or at least aiding the cleaning of said apparatus.

2. Method according to claim 1, **characterized** in supplying said solvent to said vessel (1',1'',1''') via an inlet of the vessel and introducing said cleaning fluid into the apparatus via an outlet of the vessel.

3. Method according to claim 1 or 2, **characterized** in supplying water to said vessel as said solvent.

4. Method according to claim 1, 2 or 3, **characterized** by recirculating at least a portion of said cleaning fluid present in said flow path through a recirculation path from a position downstreams in said flow path and to a position (2) adjacent said first inlet (1a).

5. Method according to claim 1, 2 or 3, wherein said apparatus comprises a heating device (2) for heating said treatment fluid, **characterized** by
recirculating at least a portion of said cleaning fluid present in said flow path through a recirculation path;
heating said cleaning fluid for increasing and essentially maintaining the temperature of said cleaning fluid at a predetermined temperature, for exampel a little over 90°C in said recirculation path, and
flowing said cleaning fluid through a non-recirculation path, wherein the temperature of said cleaning fluid is allowed to gradually decrease to a lower predetermined value, for example 80°C.

6. Method according to claim 5, **characterized** in
flowing said cleaning fluid at a high first flow speed in the recirculation path, and
flowing said cleaning fluid at a low second flow speed, for example, one fifth of the first flow speed in the non-recirculation path.

7. Method according to anyone of the preceeding claims, wherein said conditioning means of the apparatus comprises control means (5,6,9,14,19,22,23,27,31), for example throttles, included in said flow path, **characterized** by completely or partially by-passing said control means for reducing the flow-resistances thereof, during the cleaning.

8. Use of a vessel suitable for performing the method according to anyone of the preceding claims,
**characterized** by
supplying a solvent to the vessel containing a cleaning agent in concentrated liquid or powder form for mixing with said cleaning agent for diluting and/or dissolving said cleaning agent for forming a cleaning fluid;
introducing said cleaning fluid into a medical treatment apparatus for achieving or at least aiding the cleaning of said apparatus.

9. Use according to claim 8, **characterized** in that said solvent is water.

10. A vessel suitable for performing the method according to any one of claims 1 - 7, **characterized** in that said vessel comprises said cleaning agent in a quantity sufficient for one cleaning operation.

11. Vessel according to claim 10, **characterized** in that the vessel is completely sealed and is provided with penetrable membranes at its inlet and outlet.

12. Vessel according to claim 10 or 11, **characterized** in that the vessel contains one of the following concentrates: citric acid, peracetic acid, oxalic acid, sodium hydroxide, sodium hypochloride, sodium carbonate or suitable combinations thereof.

13. Vessel according to anyone of claims 10 - 12, **characterized** in that said cleaning agent is a powder and is citric acid in crystalline form.

14. Vessel according to anyone of claims 10 - 13, **characterized** in that said vessel is a cartridge.
